# EUROPÄISCHE PATENTSCHRIFT

(11) **EP 0 798 293 B1**
(45) Veröffentlichungstag und Bekanntmachung des Hinweises auf die Patenterteilung: **09.08.2000**
(21) Anmeldenummer: 97104120.7
(22) Anmeldetag: 12.03.1997
(51) Int. Cl.: C07C 279/36

(54) **Verfahren zur Herstellung von N-Methyl-N'-nitroguanidin**
Process for the preparation of N-Methyl-N'-nitroguanidine
Procédé pour la préparation de N-Methyl-N'-nitroguanidine

(30) Priorität: 25.03.1996 DE 19611654
(43) Veröffentlichungstag der Anmeldung: 01.10.1997
(73) Patentinhaber: BAYER AG, 51368 Leverkusen (DE)
(72) Erfinder: Gallenkamp, Bernd, Dr., 42113 Wuppertal (DE); Rohe, Lothar, Dr., 42113 Wuppertal (DE)

(56) Entgegenhaltungen:
- JOURNAL OF THE AMERICAN CHEMICAL SOCIETY, Bd. 69, 1947, DC US, Seiten 3028-3030, XP002033022 A.F. MCKAY ET AL.: "Preparation and properties of N-methyl-N-nitroso-N'-ntroguanidine"

## Beschreibung

Die Erfindung betrifft ein neues Verfahren zur Herstellung von N-Methyl-N'-nitroguanidin.

Es ist bekannt, daß man N-Methyl-N'-nitroguanidin erhält, wenn man zunächst 5-Methylisothiuroniumsulfat der Formel (A) in üblicher Weise nitriert und anschließend in einer zweiten Reaktionsstufe die Methylmercaptogruppe gegen Methylamin gemäß dem folgenden Reaktionsschema substituiert: 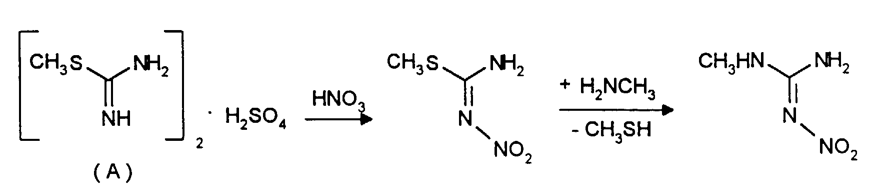 (vgl. hierzu JACS 76, 1877 (1954)).

Dieses Verfahren hat jedoch den Nachteil, daß es sich um eine zweistufige Umsetzung handelt. Wenngleich die Ausbeuten in beiden Stufen verhältnismaßig gut sind, wirft die Abspaltung von Methylmercaptan, vor allem bei einer Durchführung im großtechnischen Maßstab, verfahrenstechnische Probleme auf.

Ferner ist bekannt, daß das N-Methyl-N'-nitroguanidin erhalten werden kann, wenn man eine alkalische Lösung (Kaliumhydroxid) von Nitroguanidin mit Methylamin-Hydrochlorid bei 60°C gemäß dem folgenden Reaktionsschema umsetzt: 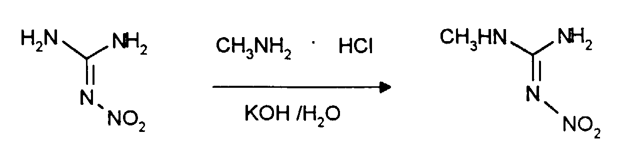 (vgl. hierzu JACS 69, 3028 (1947)).

Dieses Verfahren hat jedoch den Nachteil, daß zum Erhalt eines sauberen Produkts mindestens ein bis zwei Umkristallisationen zur Entfernung anorganischer Verunreinigungen notwendig sind, was zu merklichen Ausbeuteverlusten führt.

Es wurde nun gefunden, daß man N-Methyl-N'-nitroguanidin der Formel (I) 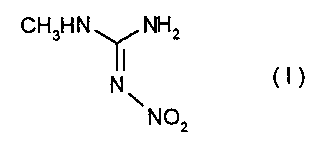 erhält,
wenn man Nitroguanidin in der Formel (II) 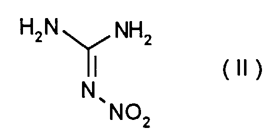 mit wäßriger Methylaminlösung bei Temperaturen zwischen 0°C und 40°C umsetzt.

Überraschenderweise kann nach dem erfindungsgemäßen Verfahren das N-Methyl-N'-nitroguanidin der Formel (I) auf einfache Art in sehr guten Ausbeuten und in hoher Reinheit erhalten werden, obwohl nach dem Stand der Technik nicht zu erwarten war, daß die Reaktion unter diesen milden Bedingungen so erfolgreich verläuft. Überraschend ist außerdem die hohe Selektivität der erfindungsgemäßen Umsetzung; es ist keine Dialkylierung zu beobachten.

Die erfindungsgemäße Umsetzung hat somit den Vorteil einer günstigeren Verfahrensdurchführung bei gleichzeitiger hoher Ausbeute. Hierbei seien im einzelnen genannt: keine Hilfsstoffe erforderlich, niedrige Reaktionstemperatur, einfache Isolierung des Endprodukts ohne gesonderte Reinigungsschritte sowie Vermeidung von Methylmercaptan.

Der Reaktionsablauf des erfindungsgemäßen Verfahrens kann durch das folgende Reaktionsschema skizziert werden:

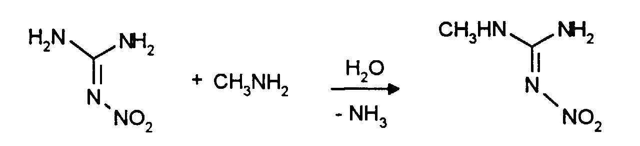

Die Ausgangsstoffe Nitroguanidin der Formel (II) und Methylamin sind allgemein bekannte Verbindungen der organischen Chemie.

Das erfindungsgemäße Verfahren wird in Gegenwart von Wasser als Verdünnungsmittel durchgeführt. Es ist aber auch möglich in organisch/wässrigen Systemen zu arbeiten, wobei alle üblichen, mit Wasser mischbaren organischen Lösungsmittel eingesetzt werden können. Beispielhaft genannt seien Ketone, wie Aceton, Methyl-ethyl-keton oder Methyl-isobutyl-keton; Nitrile, wie Acetonitril oder Propionitril sowie Alkohole, wie Methanol oder Ethanol.

Die Reaktionstemperaturen können bei der Durchführung des erfindungsgemäßen Verfahrens in einem größeren Bereich variiert werden. Im allgemeinen arbeitet man bei Temperaturen zwischen 10°C und 30°C, vorzugsweise bei Raumtemperatur.

Bei der Durchführung des erfindungsgemäßen Verfahrens setzt man pro Mol Nitroguanidin der Formel (II) im allgemeinen 1 bis 3 Mol, vorzugweise 1 bis 2 Mol Methylamin ein.

In einer besonderen Ausführungsform des erfindungsgemäßen Verfahrens werden beide Ausgangsstoffe direkt als wässrige Lösungen eingesetzt.

Die Aufarbeitung kann auf übliche Art und Weise durchgeführt werden.

Das nach dem erfindungsgemäßen Verfahren herzustellende N-Methyl-N'-nitroguanidin der Formel (I) kann als Zwischenprodukt zur Herstellung von biologisch aktiven Verbindungen, beispielsweise von Insektiziden verwendet werden (vgl. z.B. EP-A 0 376 279 und EP-A 0 428 941).

### Herstellungsbeispiele

### Beispiel 1

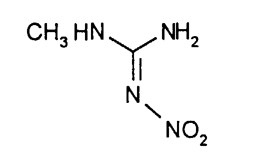

Zu einer Suspension von 138,8 g (1 Mol) Nitroguanidin (enthält ca. 25 % Wasser) in 750 ml Wasser tropft man bei 18 bis 20°C innerhalb von 10 Minuten 150 g (ca. 1,5 Mol) ca. 30 %-ige wässrige Methylaminlösung. Man läßt das Reaktionsgemisch 24 Stunden bei 20 bis 25°C nachrühren. Danach wird auf ca. 5°C abgekühlt, der Niederschlag abgesaugt, mit Mutterlauge nachgewaschen, trockengesaugt, nochmals mit Petrolether nachgewaschen und im Vakuumtrockenschrank bei 45°C getrocknet.

Man erhält 100,8 g (85,4 % der Theorie) N-Methyl-N'-nitroguanidin vom Schmelzpunkt 159°C mit einem Gehalt von 100 % (nach HPLC).

## Patentansprüche

1. Verfahren zur Herstellung von N-Methyl-N'-nitroguanidin der Formel (I) 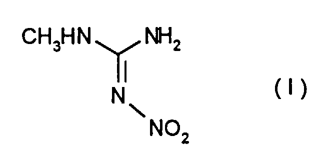 dadurch gekennzeichnet, daß man Nitroguanidin in der Formel (II) 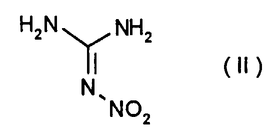 mit wäßriger Methylaminlösung bei Temperaturen zwischen 0°C und 40°C umsetzt.

## Claims

1. Process for preparing N-methyl-N'-nitroguanidine of the formula (I) 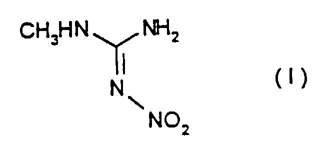 characterized in that nitroguanidine of the formula (II) 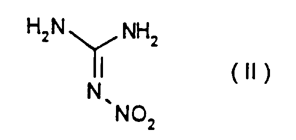 is reacted with aqueous methylamine solution at temperatures between 0°C and 40°C.

## Revendications

1. Procédé pour la préparation de la N-méthyl-N'-nitroguanidine répondant à la formule (I) 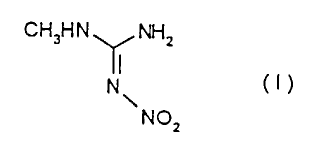 caractérisé en ce qu'on fait réagir de la nitroguanidine répondant à la formule (II) 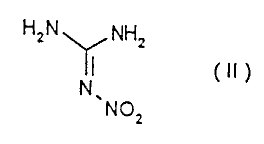 avec une solution aqueuse de méthylamine à des températures entre 0°C et 40°C.
